(19) Europäisches Patentamt
European Patent Office
Office européen des brevets

(11) Numéro de publication: **0 197 807 B1**

# (12) FASCICULE DE BREVET EUROPEEN

(45) Date de publication du fascicule du brevet: **18.04.90**

(21) Numéro de dépôt: **86400427.0**

(22) Date de dépôt: **28.02.86**

(51) Int. Cl.[4]: **C07D 498/04**, A61K 31/535 // C07D265/36 ,(C07D498/04, 265:00, 231:00)

(54) **Nouveaux dérivés de la pyrazolobenzoxazine, leurs sels, procédé et intermédiaires de préparation, application à titre de médicaments et compositions les renfermant.**

(30) Priorité: **01.03.85 FR 8503036**

(43) Date de publication de la demande: **15.10.86 Bulletin 86/42**

(45) Mention de la délivrance du brevet: **18.04.90 Bulletin 90/16**

(84) Etats contractants désignés: **AT BE CH DE FR GB IT LI LU NL SE**

(56) Documents cités:
**EP-A- 0 013 787**
**EP-A- 0 033 767**
**US-A- 4 552 956**

(73) Titulaire: **ROUSSEL-UCLAF, 35, boulevard des Invalides, F-75007 Paris(FR)**

(72) Inventeur: **Nedelec, Lucien, 45, boulevard de l'Ouest, F-93340-Le Raincy(FR)**
Inventeur: **Fauveau, Patrick, 40, avenue Camille Desmoulins, F-93190-Livry-Gargan(FR)**
Inventeur: **Hamon, Gilles, 40, rue de Bagneux, F-92120-Montrouge(FR)**
Inventeur: **Oberlander, Claude, 2, rue Paul Albert, F-75018-Paris(FR)**

(74) Mandataire: **Fritel, Hubert et al, Département des Brevets ROUSSEL UCLAF B.P. no 9, F-93230 Romainville(FR)**

EP 0 197 807 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne de nouveaux dérivés de la pyrazolobenzoxazine ainsi que leurs sels, le procédé de préparation, l'application à titre de médicaments de ces nouveaux dérivés, et les compositions les renfermant.

Des produits similaires à ceux de la présente demande ont été décrits dans le brevet américain USP 4 552 956 publié après la date prioritaire de la présente demande.

L'invention a pour objet de nouveaux dérivés de la pyrazolobenzoxazine, ainsi que leurs sels d'addition avec les acides minéraux ou organiques, caractérisés en ce qu'ils répondent à la formule générale I:

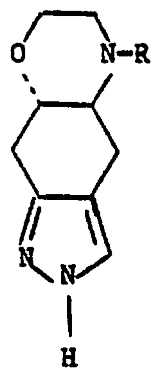

(I)

dans laquelle R représente un atome d'hydrogène ou un radical alkyle renfermant de 1 à 5 atomes de carbone, cycloalkylalkyle renfermant de 4 à 7 atomes de carbone, alkényle ou alkynyle renfermant de 3 à 5 atomes de carbone, étant entendu que la liaison multiple ne se trouve pas entre les atomes de carbone en α et en β de l'azote, ou aralkyle renfermant de 7 à 12 atomes de carbone, éventuellement substitué par un ou plusieurs radicaux choisis parmi les radicaux alkyles renfermant de 1 à 5 atomes de carbone, alkoxy renfermant de 1 à 5 atomes de carbone, amino, hydroxy et les atomes d'halogène.

Dans la formule générale I et dans ce qui suit, le terme radical alkyle renfermant de 1 à 5 atomes de carbone désigne, de préférence, un radical méthyle, éthyle, propyle, ou isopropyle; le terme radical cycloalkylalkyle renfermant de 4 à 7 atomes de carbone désigne, de préférence, un radical cyclopropylméthyle ou cyclobutylméthyle; le terme radical aralkyle renfermant de 7 à 12 atomes de carbone désigne, de préférence, un radical benzyle, phénéthyle, ou naphtylméthyle; l'aryle de l'aralkyle peut être plurisubstitué ou, de préférence monosubstitué et les substituants sont choisis dans le groupe constitué par les halogènes, les radicaux alkyle $C_1$–$C_5$, alkoxy $C_1$–$C_5$, amino et hydroxy; le terme atome d'halogène désigne, de préférence, un atome de chlore ou de brome.

Le pointillé signifie que la fonction des cycles concernés est trans.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits, objet de l'invention, on peut citer plus particulièrement les dérivés répondant à la formule (I), ainsi que leurs sels d'addition avec les acides minéraux ou organiques, caractérisés en ce que dans ladite formule (I), R représente un atome d'hydrogène ou un radical alkyle renfermant de 1 à 5 atomes de carbone.

Parmi ceux-ci, on peut citer tout particulièrement la [4a SR trans] 5-propyl 2,4,4a,5,6,7,8a,9-octahydro pyrazolo [4,3 g] 1,4-benzoxazine, ainsi que ses sels d'addition avec les acides minéraux ou organiques.

On peut citer également:

– la [4a SR trans] 5-méthyl 2,4,4a,5,6,7,8a,9-octahydro pyrazolo [4,3-g] 1,4-benzoxazine ainsi que ses sels d'addition avec les acides minéraux et organiques.

L'invention a également pour objet un procédé de préparation des dérivés, tels que définis par la formule I ci-dessus, ainsi que de leurs sels, caractérisé en ce que l'on condense l'énolate d'une cétone de formule II:

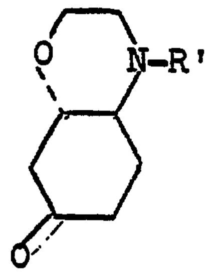

II

dans laquelle R′ a la signification de R déjà indiquée, à l'exception de l'hydrogène, avec un formiate d'alkyle de formule III:

HCOO-Alk III

dans laquelle Alk représente un radical alkyle renfermant de 1 à 4 atomes de carbone, pour obtenir un produit de formule IV:

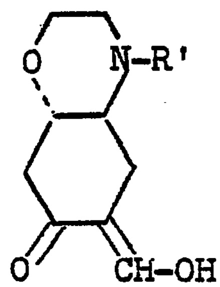

IV

dans laquelle R′ a la signification déjà indiquée, que l'on fait réagir avec l'hydrazine pour obtenir un produit de formule $I_A$:

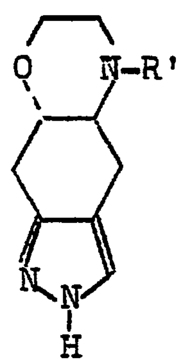

$I_A$

dans laquelle R′ a la signification indiquée, que soit l'on isole et, si désiré, salifie, soit, dans le cas où R′ représente un radical benzyle, l'on soumet à une hydrogénation catalytique pour obtenir un produit de formule $I_B$:

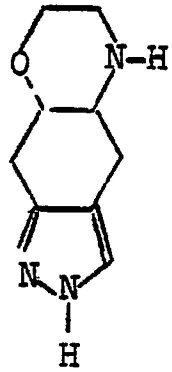

$I_B$

que soit l'on isole et, si désiré, salifie, soit l'on fait réagir avec un halogénure de formule V:

Hal R″ V

dans laquelle Hal représente un atome de chlore, de brome ou d'iode, et R″ a la signification de R déjà indiquée à l'exception de l'hydrogène et du radical méthyle, pour obtenir un produit de formule $I_C$:

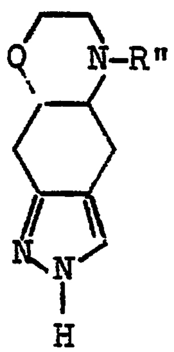

$I_C$

dans laquelle R″ a la signification déjà indiquée, que l'on isole et, si désiré, salifie.

La formation de l'énolate de la cétone de formule II est réalisée de préférence, en présence d'un hydrure alcalin tel que l'hydrure de sodium, ou d'un alcoolate, tel que l'éthylate de sodium ou le terbutylate de potassium, notamment en présence d'une quantité catalytique d'un alcool aliphatique tel que le méthanol ou l'éthanol.

Le formiate d'alkyle est, par exemple, un formiate de méthyle ou de propyle, et de préférence d'éthyle.

L'hydrazine est utilisée, de préférence, sous forme d'hydrate d'hydrazine.

L'hydrogénation catalytique est réalisée, par exemple, en présence d'un catalyseur tel que le nickel, ou, de préférence le palladium.

La réaction du dérivé de vormule $I_B$ avec l'halogénure de formule V est réalise, de préférence, en présence d'une base telle qu'un carbonate ou bicarbonate alcalin comme le carbonate de sodium, un hydroxyde comme la soude ou la potasse, un alcoolate comme l'éthylate de sodium, ou une amine tertiaire comme la triéthylamine ou la pyridine.

L'halogénure de formule V est, par exemple, un bromure ou un chlorure, et de préférence un iodure.

Dans une variante du procédé ci-dessus décrit, l'on fait réagir la cétone de formule II avec le diméthylacetal du diméthylformamide au reflux d'un solvant à point d'ébullition de 80°C à 130°C, puis traite à l'aide d'hydrazine pour obtenir un produit de formule $I_A$ que, si désiré, l'on traite comme indiqué ci-dessus.

Le solvant à point d'ébullition de 80°C à 130°C est, par exemple, le benzène, le toluène ou le xylène.

Pour fixer un radical R différent de l'hydrogène sur le dérivé de formule $I_B$, on peut opérer également par action du dérivé de formule $I_B$, avec un dérivé (selon le cas, un aldéhyde ou une cétone) de formule

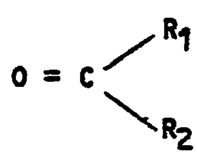

dans laquelle $R_1$ et $R_2$ sont tels que:

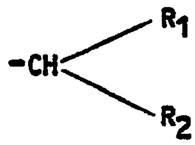

a la signification de R déjà indiquée, à l'exception de l'hydrogène, en présence d'un agent de réduction (borohydrure ou cyanoborohydrure alcalin, tel que borohydrure ou cyanoborohydrure de sodium, ou hydrogénation catalytique). C'est ainsi, par exemple, que si le dérivé de formule $O{=}CR_1R_2$ est le formaldéhyde ou l'acétaldéhyde, on obtient respectivement pour R les valeurs méthyle et éthyle.

Les produits de formule II peuvent être préparés à partir d'un produit de formule VI, dont on protège l'hydroxyle:

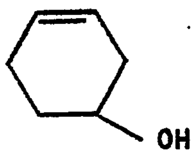

VI

pour obtenir un produit de formule VII:

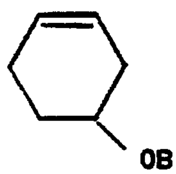

VII

dans laquelle B représente un groupement protecteur classique de la fonction hydroxyle, que l'on fait réagir avec un peracide pour obtenir un produit de formule:

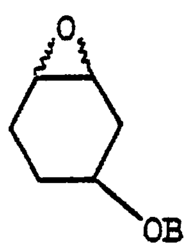

VIII

dans laquelle B a la signification déjà indiquée et les traits ondulés signifient que la conformation est syn ou anti, fait réagir l'isomère anti avec une amine de formule IX:

$$R'-NH_2 \qquad IX$$

dans laquelle R' a la signification déjà indiquée, pour obtenir les isomères de formules Xa et Xb:

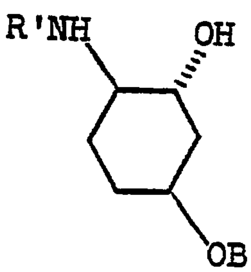

Xa

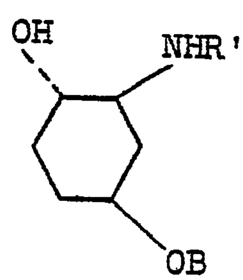

Xb

dans laquelle R′ et B ont la signification déjà indiquée, isole l'isomère Xa et le fait réagir avec un halogénure d'hydroxy éthyle pour obtenir un produit de formule XI:

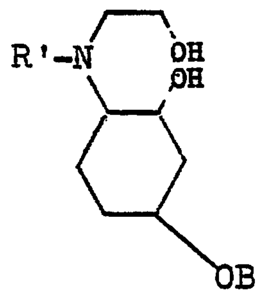

XI

dans laquelle B et R′ ont la signification déjà indiquée, que l'on cyclise pour obtenir un produit de formule XII:

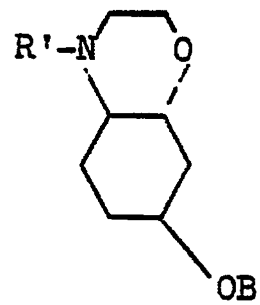

XII

dans laquelle B et R′ ont la signification déjà indiquée, que l'on soumet à l'action d'un agent de déblocage de l'hydroxyle pour obtenir un produit de formule XIII:

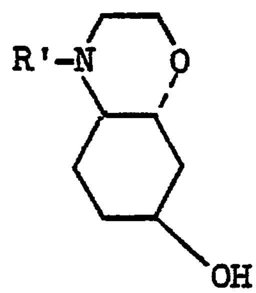

XIII

dans laquelle R′ a la signification déjà indiquée, que l'on oxyde pour obtenir de produit de formule II cherché.

Le groupement protecteur classique de la fonction hydroxyle est, par exemple un reste silylé tel que le diméthyl tertbutylsilyle, le triméthyl silyle ou le diphénylméthyl silyle, ou un ester tel qu'un benzoate. Pour le greffer, on opère, de préférence, à l'aide de l'halogénure silylé correspondant en présence de diméthylamino pyridine dans le cas d'un éther silylé ou à l'aide d'un halogénure de benzoyle en présence d'un agent de condensation tel que ceux décrits ci-dessus, dans le cas d'un radical benzoyle.

Le peracide utilisé est, par exemple, l'acide peracétique ou l'acide pertrifluoroacétique et, de préférence, l'acide m-chloro perbenzoïque. On opère, de préférence, en présence d'une base telle qu'un carbonate alcalin.

La réaction de l'époxyde de formule VIII avec l'amine de formule IX est réalisée de préférence au reflux d'un alcanol tel que le méthanol ou l'éthanol.

L'halogénure d'hydroxyéthyle est, par exemple, un chlorure ou un bromure, et de préférence un iodure. On le fait avantageusement réagir en présence d'un carbonate alcalin ou d'une amine tertiaire.

La cyclisation du produit de formule XI est réalisée, de préférence, par activation de l'hydroxyle primaire, à l'aide, par exemple, du complexe N-chlorodiisopropylamine – Tris diméthylamino phosphine, à basse température, aux environs de –40°C.

Le déblocage de l'hydroxyle est réalisé dans les conditions habituelles utilisées selon l'agent de blocage choisi, c'est-à-dire, par exemple, l'action du tétrabutylammonium ou le reflux d'un solvant tel que le benzène, le toluène ou le xylène en présence d'un acide tel que l'acide paratoluène sulfonique, l'acide sulfonique ou encore l'action du mélange acide acétique-tétrahydrofuranne-eau dans le cas d'un éther silylé.

Dans le cas du benzoate, on opère à l'aide d'une base telle qu'un hydroxyde alcalin, comme la soude ou la potasse.

L'oxydation du produit de formule XIII est réalisée, par exemple, à l'aide de dichromate de pyridine, le chlorochromate de pyridine, ou de préférence à l'aide d'un mélange anhydride chromique-acide sulfurique-eau (réactif de Helbron-Jones).

La séparation des isomères peut être effectuée à l'un ou l'autre des stades, par les moyens classiques, notamment par chromatographie.

Les dérivés de formule I présentent un caractère basique. On peut avantageusement préparer les sels d'addition des dérivés de formule I, en faisant réagir, en proportions sensiblement stœchiométriques, un acide minéral ou organique avec ledit dérivé de formule I. Les sels peuvent être préparés sans isoler les bases correspondantes.

Les dérivés, objet de la présente invention, possèdent de très intéressantes propriétés pharmacologiques; ils sont doués notamment de remarquables propriétés agonistes dopaminergiques, hypotensives et antihypertensives. Ils sont, par ailleurs, doués de propriétés antianoxiques.

Ces propriétés sont illustrées plus loin dans la partie expérimentale.

Ces propriétés justifient l'utilisation des dérivés de la pyrazolobenzoxazine, ainsi que de leurs sels pharmaceutiquement acceptables, à titre de médicaments.

La présente demande a ainsi également pour objet l'application, à titre de médicaments, des dérivés de la pyrazolobenzoxazine, tels que définis par la formule I, ainsi que de leurs sels d'addition avec les acides pharmaceutiquement acceptables.

Parmi les médicaments objet de l'invention on retient, de préférence, les médicaments caractérisés en ce qu'ils sont constitués par les nouveaux dérivés de la pyrazolobenzoxazine répondant à la formule I, dans laquelle R représente un atome d'hydrogène ou un radical alkyle renfermant de 1 à 5 atomes de carbone, ainsi que par leurs sels d'addition avec les acides pharmaceutiquement acceptables.

Parmi ces derniers, on retient tout particulièrement la [4a <u>SRtrans</u>] 5-propyl 2,4,4a,5,6,7,8a,9-octahydro pyrazolo [4,3-g] 1,4-benzoxazine, ainsi que ses sels d'addition avec les acides pharmaceutiquement acceptables.

Les médicaments selon l'invention trouvent leur emploi, par exemple, dans le traitement des syndromes neurologiques d'origine extrapyramidale, par exemple dans le traitement de la maladie de Parkinson et dans le traitement des syndromes parkinsonniens post-encéphalitiques. Ils peuvent aussi être utilisés dans le traitement de l'hypertension artérielle essentielle, de l'hypertension de la cinquantaine, de la ménopause, du diabétique, de l'obèse et du pléthorique, ainsi que dans le traitement de l'hypertension artérielle du sujet âgé, ou atteint d'artériosclérose, et dans le traitement de l'hypertension d'origine rénale. Ils peuvent aussi être utilisés dans le traitement de la sénescence cérébrale ou des manifestations liées à une hypoxie cérébrale.

La dose usuelle, variable selon le dérivé utilisé, le sujet et l'affection en cause peut être, par exemple, de 1 mg à 200 mg par jour. Par voie orale, chez l'homme, le dérivé de l'exemple 3 peut être administré à la dose quotidienne de 1 mg à 100 mg, par exemple, pour le traitement de la sénescence cérébrale, soit environ de 0,015 mg à 1,5 mg par kilogramme de poids corporel.

L'invention a également pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule I et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les exemples qui suivent illustrent la présente invention sans toutefois la limiter.

Exemple no 1: [4a SR trans] 2,4,4a,5,6,7,8a,9-octahydro (phénylméthyl) pyrazolo [4,3-g] 1,4-benzoxazine.

<u>Stade A</u>: [4a SR <u>trans</u>] 6-hydroxy méthylène octahydro 4-(phénylméthyl) 7 H-1,4-benzoxazin-7-one.

On place sous agitation et atmosphère inerte 1,42 g d'hydrure de sodium à 50% dans 50 cm3 d'éther, avec 4,6 cm3 de formiate d'éthyle et 0,2 cm3 d'éthanol, ajoute goutte à goutte en 30 mn une solution de 3,5 g de [4a SR <u>trans</u>] octahydro 4-phénylméthyl 7 H-1,4-benzoxazin-7-one dans 40 cm3 d'éther, laisse

pendant 1H 30 sous agitation, ajoute de la glace, acidifie à l'aide d'une solution aqueuse 2N d'acide chlorhydrique, décante et amène la phase aqueuse à pH = 5 à l'aide de solution aqueuse 2N de soude. On extrait au chlorure de méthylène, lave à l'eau, sèche et amène à sec sous pression réduite, obtient 3,6 g d'un produit que l'on purifie par chromatographie sur silice (éluant: benzène-acétate d'éthyle 7–3), pour récupérer 2,7 g de l'isomère majoritaire sous forme d'une huile jaune,
Spectre UV (Ethanol) pour PM = 273,31

| | | | |
|---|---|---|---|
| max | 278 nm | $E^1_1 = 298$ | $\varepsilon = 8100$ |
| inflexion | 311 nm | $E^1_1 = 111$ | |
| inflexion | 342 nm | $E^1_1 = 29$ | |

Spectre RMN dans CDCl3 à 250 MHz

| | | | |
|---|---|---|---|
| pic | à | 14,3 ppm | H de l'hydroxyle |
| | | 8,70 ppm | H de = CH en position 6 |
| pics | à | 2,45 et 2,75 ppm | H de = CH en position 8 |
| pic | à | 3,55 ppm | H de = CH en position 8a |
| pics | à | 3,67 et 3,80 ppm | H de = CH en position 2 |
| pics | de | 7,2 à 7,35 ppm | H du phényle |
| pics | à | 3,16 et 4,15 ppm | H en α du phényle |
| pics | de | 2,2 à 3,0 ppm | autres hydrogènes |

Stade B: [4a SR trans] 2,4,4a,5,6,7,8a,9-octahydro 5-(phénylméthyl) pyrazolo [4,3-g] 1,4-benzoxazine.

On place pendant 2 heures sous agitation et atmosphère inerte 2,85 g du produit du stade A dans 25 cm3 d'éthanol, avec 1 cm3 d'hydrate d'hydrazine, amène à sec sous pression réduite, purifie par chromatographie sur silice (Eluant: chlorure de méthylène-méthanol 97–3) et obtient 2,2 g du produit attendu F 145°C après recristallisation dans l'acétone.

Analyse pour $C_{16}H_{19}N_3O$ = 269,33

| | | | | | | |
|---|---|---|---|---|---|---|
| Calculé: | C% | 71,35 | H% | 7,11 | N% | 15,60 |
| Trouvé: | | 71,1 | | 7,2 | | 15,3 |

Exemple 2: [4a SR trans] 2,4,4a,5,6,7,8a,9-octahydro pyrazolo [4,3-g] 1,4-benzoxazine et son méthane sulfonate.

On hydrogène à saturation 3,4 g de produit de l'exemple 1 dans 100 cm3 de méthanol en présence de 941 mg de palladium à 9,6% sur charbon actif, filtre, rince à l'éthanol, évapore le filtrat sous pression réduite et obtient 2,13 g de la base attendue F≈214°C. Formation du méthane sulfonate.

On dissout 720 mg de base ci-dessus dans 10 cm3 de méthanol, ajoute 0,26 cm3 d'acide méthane sulfonique, laisse cristalliser, filtre, sèche sous pression réduite et obtient 776 mg du produit attendu F≈262°C.

Analyse pour $C_{10}H_{16}N_3O_4S$ = 275,328

| | | | | | | | | |
|---|---|---|---|---|---|---|---|---|
| Calculé: | C% | 43,62 | H% | 6,22 | N% | 15,26 | S% | 11,64 |
| Trouvé: | | 43,7 | | 6,3 | | 15,3 | | 11,6 |

Exemple 3: [4a SR trans] 5-propyl 2,4,4a,5,6,7,8a,9-octahydro pyrazolo [4,3-g] 1,4-benzoxazine et son oxalate.

On place pendant 2 heures sous agitation à 60°C 1,14 g du produit de l'exemple 2 dans 10 cm3 de diméthylformamide, avec 0,63 cm3 d'iodure de propyle en présence de 1,06 g de carbonate de potassium, refroidit, ajoute de l'eau, extrait à plusieurs reprises au chlorure de méthylène, sèche, amène à sec sous pression réduite, purifie par chromatographie sur silice (éluant chlorure de méthylène-méthanol (95–5)), et obtient 1,08 g du produit attendu.

Formation de l'oxalate

On ajoute une solution de 488 mg d'acide oxalique dans 5 cm3 de méthanol à une solution de 1,2 g de la

base ci-dessus dans 10 cm3 de méthanol, laisse cristalliser, filtre, lave au méthanol, recristallise dans le méthanol et obtient 1,04 g du produit attendu F≈220°C.

Analyse pour $C_{14}H_{21}N_3O_5$ = 311,33

| | | | | | | |
|---|---|---|---|---|---|---|
| Calculé: | C% | 54,01 | H% | 6,80 | N% | 13,49 |
| Trouvé: | | 54,1 | | 6,9 | | 13,6 |

Préparation du [4a SR trans] octahydro 4-(phénylméthyl) 7H-1,4-benzoxazine-7-one utilisée au départ de l'exemple 1.

Stade A: (3-cyclohexen-1-oxy) diméthyl (1,1-diméthyléthyl) silane.

On place sous agitation et atmosphère inerte 10 g de 3-cyclohexen-1-ol en solution dans 100 cm3 de chlorure de méthylène, avec 12 g de 4-diméthylaminopyridine et 17 cm3 de triéthylamine, ajoute goutte à goutte en 1 heure 15 g de chlorure de tertbutyl diméthyl silyle, laisse pendant 1 heure sous agitation, filtre, lave le filtrat à l'aide d'acide chlorhydrique 2N, puis à l'eau, sèche et amène à sec sous pression réduite.

Par purification et par chromatographie sur silice (Eluant: cyclohexane - acétate d'éthyle-7-3) on obtient 19,5 g du produit attendu.

Stade B: diméthyl (1,1-diméthylêthyl) / [1SR(1α,3β,6α)7-oxabicyclo] 4,1,0 [heptan-3-yl oxy] silane.

On place sous agitation et sous atmosphère inerte 17 g du produit du stade précédent et 10,2 g de carbonate de sodium dans 200 cm3 de chlorure de méthylène et refroidit à 0°—+5°C.

On ajoute goutte à goutte 20,6 g d'acide m-chloro perbenzoïque à 80% en solution dans 200 cm3 de chlorure de méthylène et laisse sous agitation à environ 5°C pendant 16 heures. On filtre, le précipité formé, le lave au chlorure de méthylène, lave le filtrat avec une solution de thiosulfate de sodium, sèche et amène à sec sous pression réduite. On purifie par chromatographie sur silice, recueille la première fraction éluée (éluant: benzène) correspondant à l'époxyde anti désiré et obtient 5,8 g du produit attendu.

Stade C: diméthyl (1,1-diméthyléthyl) / [1 RS (1α,3β,4α) 3-hydroxy 4-](phénylméthyl) amino[cyclohexyloxy] silane.

On porte au reflux pendant 16 heures, sous agitation et atmosphère inerte 7,3 g du produit obtenu comme au stade B dans 70 cm3 de méthanol avec 16 cm3 de benzyl amine. On amène à sec sous pression réduite, purifie par chromatographie sur silice (Eluant: benzène-acétate d'éthyle 5–5) et récupère 4 g du produit attendu correspondant à la fraction la plus mobile, F≈65°C après recristallisation dans l'hexane.

Stade D: diméthyl (1,1-diméthyléthyl) [(1 SR (1α,3β,4α) 3-hydroxy-4](2-hydroxyéthyl) phénylméthylamino [1-cyclohexyloxy] silane.

On place pendant 16 heures à 70°C, sous agitation et atmosphère inerte, 16 g du produit du stade C dans 100 cm3 de diméthylformamide avec 13,1 g de carbonate de potassium et 28 cm3 de bromoéthanol. On verse sur de la glace, extrait plusieurs fois au chlorure de méthylène, lave à l'eau, sèche, amène à sec sous pression réduite, purifie par chromatographie sur silice (Eluant: Benzène-Acétate d'éthyle 5–5) et obtient 14,8 g du produit attendu F≈88°C après recristallisation dans l'hexane.

Stade E: diméthyl (1,1-diméthyléthyl) [4a SR (4aα,7β,8aβ) octahydro 2H-1,4-benzoxazin-7 yloxy] silane.

On ajoute goutte à goutte à environ –40°C, sous atmosphère inerte et agitation, 18,6 cm3 de tris diméthylaminophosphine à 14,4 g de produit du stade D en solution dans 250 cm3 de chlorure de méthylène avec 12,8 g de N-chlorodiisopropylamine. On laisse revenir à température ambiante, ajoute de l'eau, extrait à plusieurs reprises au chlorure de méthylène, sèche, amène à sec sous pression réduite, purifie par chromatographie sur silice (Eluant: benzène-acétate d'éthyle 9–1), et obtient 12,3 g du produit attendu.

Stade F: [4a SR (4aα,7β,8aβ)] octahydro 4-phénylméthyl 2H-1,4-benzoxazin-7-ol.

On place pendant 16 heures, sous agitation et atmosphère inerte, 11,9 g de produit du stade E dans 100 cm3 de tétrahydrofuranne avec 66 cm3 d'une solution molaire de fluorure de tétrabutyl ammonium dans le tétrahydrofuranne. On verse dans de l'eau, extrait à plusieurs reprises au chlorure de méthylène, sèche, amène à sec sous pression réduite, purifie par chromatographie sur silice (Eluant: chlorure de méthylène-méthanol 95–5), et obtient 6,2 g du produit attendu.

Stade G: [4a SR trans] octahydro 4-phénylméthyl 7H-1,4-benzoxazin-7-one.

On ajoute goutte à goutte, sous agitation, à environ 0°C, 12 cm3 de réactif d'Helbron Jones (anhydride chromique 270 g, acide sulfurique concentré 230 cm3, eau pour compléter à 1 litre) à une solution de 6 g de produit du stade F dans 60 cm3 d'acétone, laisse 1 H 30 sous agitation. On ajoute de la glace, refroidit à 0°C, alcalinise à l'aide d'une solution aqueuse 2N de soude, extrait à plusieurs reprises au chlorure de méthylène, lave à l'eau, sèche, amène à sec sous pression réduite, purifie par chromatographie sur silice (Eluant: chlorure de méthylène-Méthanol 95–5) et obtient 4,14 g du produit attendu sous forme d'une huile incolore.

Exemple 4:

On a préparé des comprimés répondant à la formule:
– méthane sulfonate de [4a SR trans] 2,4,4a,5,6,7,8a,9-octahydro pyrazolo [4,3-g] 1,4-benzoxazine ............. 10 mg;
– Excipient q. s. pour un comprimé terminé à .............. 100 mg.
(Détail de l'excipient: lactose, amidon, talc, stéarate de magnésium).

Exemple 5:

On a préparé des comprimés répondant à la formule:
– oxalate de [4a SR trans] 5-propyl 2,4,4a,5,6,7,8a,9-octahydro pyrazolo [4,3-g] 1,4-benzoxazine de ........ 5 mg;
– Excipient q. s. pour un comprimé terminé à ........................ 100 mg.
(Détail de l'excipient: lactose, amidon, talc, stéarate de magnésium).

Etude pharmacologique.

1. Comportement de rotation après lésion unilatérale du faisceau nigrostriatal par la 6-hydroxydopamine.

Technique:

Des rats mâles de 220 g environ sont lésés dans le faisceau dopaminergique nigrostrié, selon la méthode d'Ungerstedt (U. Ungerstedt, Acta physiol. Scand. 1971, 82, suppl. 367, 69–93) modifiée, par injection unilatérale de 9,2 µg de chlorhydrate de 6-hydroxydopamine en solution dans 4 µl de soluté physiologique contenant 0,5 mg/ml d'acide ascorbique.

Les produits étudiés sont administrés par voie intrapéritonéale. Les animaux traités, au nombre de 8 en moyenne, sont placés individuellement dans un rotomètre qui permet de compter le nombre de rotations effectuées par chaque animal dans le sens opposé au côté lésé.

Dans les conditions de l'expérience, les résultats suivants ont été obtenus:

Dès la dose de 0,05 mg/Kg, le produit de l'exemple 3 induit des rotations contralatérales au côté lésé. Il exerce donc une action agoniste des récepteurs dopaminergiques au niveau central.

2. Comportements stéréotypés.

Les essais sont réalisés sur des lots de 5 rats mâles de 150 à 180 g. Chaque animal est placé individuellement dans une cage grillagée (29 x 25 x 17 cm) contenant quelques débris de frisure de bois.

Le produit étudié est administré par voie intrapéritonéale.

Le comportement des animaux est noté de demi-heure en demi-heure pendant 5 heures avec la cotation préconisée par HALLIWELL et Coll. (Brit. J. Pharmacol. 1964, 23, 330–350).

L'animal est endormi (0), il est éveillé, mais immobile (1), il tourne dans la cage (2), il en renifle le couvercle (3), il en lèche les parois (4), il touche les copeaux ou les barreaux de la cage avec les dents (5), il mord les copeaux ou les barreaux de la cage (6).

La somme des scores par lot, relevés à différents délais après l'administration du produit étudié est déterminée.

Dès la dose de 0,1 mg/Kg, le produit de l'exemple 3 induit des mouvements stéréotypés. Il exerce donc une action agoniste dopaminergique au niveau central.

3. Détermination de l'activité hypotensive.

L'activité hypotensive a été étudiée sur des rats mâles de souche WISTAR pesant 300 g environ et anestésiés au nembutal (50 mg/Kg par voie intrapéritonéale).

Le produit testé a été administré par voie intraveineuse dans la veine jugulaire.

La pression artérielle carodienne a été mesurée avant et après administration du produit testé.

Le tableau ci-après indique les variations exprimées en pourcentage de la pression artérielle après administration du produit testé par rapport à la pression artérielle témoin initiale.

| Produit de l'exemple | Dose mg/kg | Variation % de la pression artérielle | |
|---|---|---|---|
| | | 5 minutes après l'administration | 30 minutes après l'administration |
| 3 | 0,1 | –26 | –21 |
| | 0,01 | –19 | –8 |

On peut également noter une bradycardie.

Ces effets hypotenseurs et bradycardisants sont bloqués par le sulpiride; ils sont donc d'origine dopaminergique.

4. Détermination de l'activité antihypertensive.

L'action antihypertensive a été étudiée sur des rats mâles spontanément hypertendus (souche OKAMOTO) agés de 20 semaines pesant 300 à 320 g.

Le produit a été administré par voie orale 48 heures après la pose d'un cathéter intracarotidien servant à la mesure de la pression artérielle.

La pression a été mesurée avant et 1 heure après l'administration du produit.

Pour le produit de l'exemple n$^o$ 3, à la dose de 0,5 mg/Kg, en pourcentage de la pression artérielle, après administration du produit, par rapport à la pression témoin initiale est de –15% 1 heure après l'administration.

5. Activité antianoxique

a – Test de l'anoxie hypobare:

On utilise des souris mâles d'un poids de 20–22 g, à jeun depuis cinq heures, réparties par groupe de 10 animaux.

On administre aux animaux le produit à tester, par voie sous-cutanée. Quinze minutes après administration du produit, on place les animaux dans un dessicateur de 2 litres dans lequel on amène rapidement la pression à 90 mm de Hg, au moyen d'une pompe, et note leur temps de survie exprimé en secondes.

On note l'augmentation du temps de survie, exprimé en pourcentage, des animaux traités par rapport aux animaux témoins, soumis aux mêmes conditions.

On a obtenu les résultats suivants:

| Produit de l'exemple | Doses mg/kg per os | Augmentation du temps de survie |
|---|---|---|
| 3 | 0,1 | +90% |
| | 0,01 | +37% |

b – test de l'énolase

Les cellules neuronales cérébrales en souffrance libèrent de l'énolase. Le test est réalisé chez des rats dont le cerveau a été totalement ischémié par ligature des artères irrigant le cerveau. Le produit de l'exemple n$^o$ 3 administré par voie intrapéritonéale diminue la libération d'énolase à la dose de 0,1 mg/Kg. On considère donc qu'il protège les cellules cérébrales contre l'ischémie.

6. Etude de la toxicité aiguë.

On a évalué la dose létale $DL_0$ du produit de l'exemple n$^o$ 3 après administration par voie orale chez la souris.

On appelle $DL_0$ la dose maximale ne provoquant aucune mortalité en 8 jours.

Le résultat obtenu est le suivant:

| Produit de l'exemple | $DL_0$ en mg/kg |
|---|---|
| 3 | ≥200 |

**Revendications**

1. Les dérivés de la pyrazolobenzoxazine, ainsi que leurs sels d'addition avec les acides minéraux ou organiques, caractérisés en ce qu'ils répondent à la formule générale I:

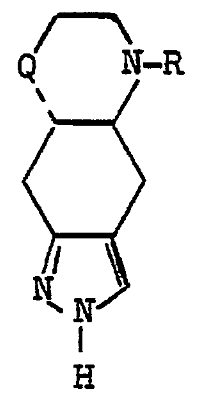

I

dans laquelle R représente un atome d'hydrogène ou un radical alkyle renfermant de 1 à 5 atomes de carbone, cycloalkylalkyle renfermant de 4 à 7 atomes de carbone, alkényle ou alkynyle renfermant de 3 à 5 atomes de carbone, étant entendu que la liaison multiple ne se trouve pas entre les atomes de carbone en α et en β de l'azote, ou aralkyle renfermant de 7 à 12 atomes de carbone, éventuellement substitué par un ou plusieurs radicaux choisis parmi les radicaux alkyle renfermant de 1 à 5 atomes de carbone, alkoxy renfermant de 1 à 5 atomes de carbone, amino, hydroxy et les atomes d'halogène.

2. Les dérivés de la pyrazolobenzoxazine tels que définis à la revendication 1, ainsi que leurs sels d'addition avec les acides minéraux ou organiques, caractérisés en ce que R représente un atome d'hydrogène ou un radical alkyle renfermant de 1 à 5 atomes de carbone.

3. Le dérivé de formule I selon la revendication 1 dont le nom suit: [4a SR trans] 5-propyl 2,4,4a,5,6,7,8a,9-octahydro pyrazolo [4,3 g] 1,4-benzoxazine, ainsi que ses sels d'addition avec les acides minéraux ou organiques.

4. Procédé de préparation des dérivés de la pyrazolobenzoxazine tels que définis par la formule I de la revendication 1, ainsi que de leurs sels, caractérisé en ce que l'on condense l'énolate d'une cétone de formule II:

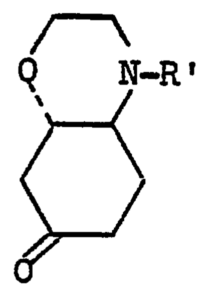

II

dans laquelle R' a la signification de R déjà indiquée, à l'exception de l'hydrogène, avec un formiate d'alkyle de formule III:

HCOO Alk III

dans laquelle Alk représente un radical alkyle renfermant de 1 à 4 atomes de carbone, pour obtenir un produit de formule IV:

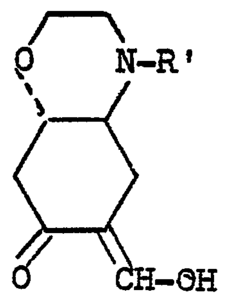

IV

dans laquelle R′ a la signification déjà indiquée, que l'on fait réagir avec l'hydrazine pour obtenir un produit de formule $I_A$:

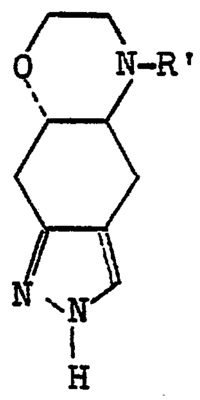

$I_A$

dans laquelle R′ a la signification indiquée, que soit l'on isole et, si désiré, salifie, soit, dans le cas où R′ représente un radical benzyle, l'on soumet à une hydrogénation catalytique pour obtenir un produit de formule $I_B$:

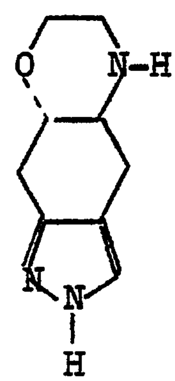

$I_B$

que soit l'on isole et, si désiré, salifie, soit l'on fait réagir avec un halogénure de formule V:

Hal R″ V

dans laquelle Hal représente un atome de chlore, de brome ou d'iode, et R″ a la signification de R déjà indiquée à l'exception de l'hydrogène et du radical méthyle, pour obtenir un produit de formule $I_C$:

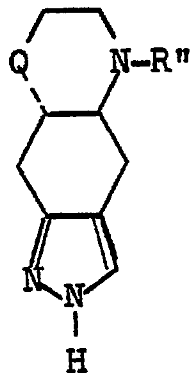

$I_C$

dans laquelle R″ a la signification déjà indiquée, que l'on isole et, si désiré, salifie.

5. Procédé selon la revendication 4, caractérisé en ce que:
- le formiate d'alkyle est le formiate d'éthyle;
- l'hydrazine est utilisée sous forme d'hydrate d'hydrazine;
- le catalyseur d'hydrogénation est le palladium;
- l'halogénure de formule V est un iodure.

6. Variante du procédé selon la revendication 4, caractérisée en ce que l'on fait réagir la cétone de formule II avec le diméthylacétal du diméthylformamide au reflux d'un solvant à point d'ébullition de 80°C à 130°C, puis traite à l'aide d'hydrazine pour obtenir un produit de formule $I_A$ que, si désiré, l'on traite comme indiqué ci-dessus.

7. Médicaments, caractérisés en ce qu'ils sont constitués par les nouveaux dérivés de la pyrazolobenzoxazine, tels que définis par la formule I de la revendication 1, ainsi que par leurs d'addition avec les acides pharmaceutiquement acceptables.

8. Médicaments, caractérisés en ce qu'ils sont constitués par les nouveaux dérivés de la pyrazolobenzoxazine, tels que définis à la revendication 2, ainsi que par leurs sels d'addition avec les acides pharmaceutiquement acceptables.

9. Médicaments, caractérisés en ce qu'ils sont constitués par le dérivé de la pyrazolobenzoxazine, tel que défini à la revendication 3, ainsi que par ses sels d'addition avec les acides pharmaceutiquement acceptables.

10. Compositions pharmaceutiques, caractérisées en ce qu'elles renferment, à titre de principe actif, l'un au moins des médicaments, tels que définis à l'une des revendications 7, 8 ou 9.

**Claims**

1. The derivatives of pyrazolobenzoxazine, as well as their addition salts with mineral or organic acids, characterised in that they correspond to the general formula I:

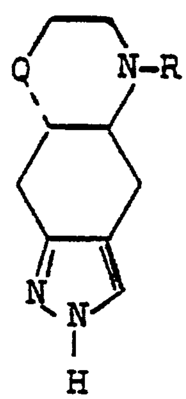

I

in which R represents a hydrogen atom or an alkyl radical containing from 1 to 5 carbon atoms, cycloalkylalkyl radical containing from 4 to 7 carbon atoms, alkenyl or alkynyl radical containing from 3 to 5 carbon atoms, it being understood that the multiple bond is not found between the carbon atoms at position alpha and at position beta of nitrogen, or aralkyl containing from 7 to 12 carbon atoms, optionally substituted by one or more radicals chosen from the following radicals; alkyl containing from 1 to 5 carbon atoms alkoxy containing from 1 to 5 carbon atoms, amino, hydroxy and the halogen atoms.

2. The derivatives of pyrazolobenzoxazine as defined in claim 1, as well as their addition salts with mineral or organic acids, characterised in that R represents a hydrogen atom or an alkyl radical containing from 1 to 5 carbon atoms.

3. The derivative of formula I according to claim 1, the name of which follows: [4a-SR-trans]-5-propyl-2,4,4a,5,6,7,8a,9-octahydro-pyrazolo-[4,3-g]-1,4-benzoxazine, as well as its addition salts with mineral or organic acids.

4. Preparation process of the derivatives of pyrazolobenzoxazine as defined by formula I in claim 1, as well as their salts, characterised in that the enolate of a ketone of formula II:

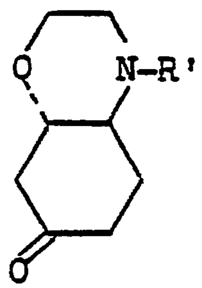

II

in which R′ has the significance of R already indicated, with the exception of the hydrogen, is condensed with an alkyl formate of formula III:

HCOO Alk III

in which Alk represents an alkyl radical containing from 1 to 4 carbon atoms, to obtain a product of formula IV:

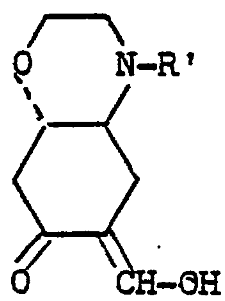

IV

in which R′ has the significance already indicated, which is reacted with hydrazine to obtain a product of formula $I_A$:

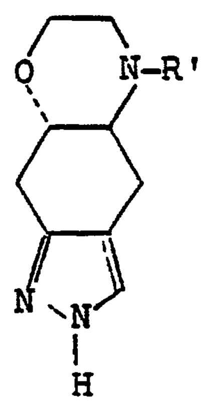

$I_A$

in which R′ has the significance indicated, which is either isolated and if desired salified, or in the case where R′ represents a benzyl radical it is subjected to a catalytic hydrogenation to obtain a product of formula $I_B$:

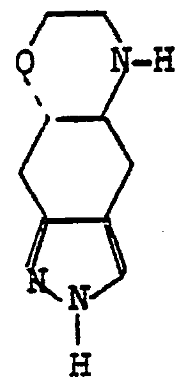

$I_B$

which is either isolated and if desired salified, or reacted with a halide of formula V:

Hal R″ V

in which Hal represents an atom of chlorine, bromine or iodine, and R″ has the significance already indicated for R with the exception of the hydrogen and the methyl radical, to obtain a product of formula $I_C$:

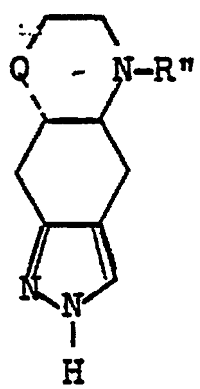

$I_C$

in which R″ has the significance already indicated, which is isolated and if desired salified.

5. Process according to claim 4, characterised in that:
- the alkyl formate is ethyl formate;
- the hydrazine is used in the form of hydrazine hydrate;
- the hydrogenation catalyst is palladium;
- the halide of formula V is an iodide.

6. A variation on the process according to claim 4, characterised in that the ketone of formula II is reacted with dimethylacetal of dimethylformamide under reflux using a solvent with a boiling point of 80°C to 130°C, then treated with hydrazine to obtain a product of formula $I_A$, which, if desired, is treated as indicated above.

7. Medicaments, characterised in that they are composed of the new derivatives of pyrazolobenzoxazine, as defined by formula I of claim 1, as well as by their addition salts with pharmaceutically acceptable acids.

8. Medicaments, characterised in that they are composed of the new derivatives of pyrazolobenzoxazine, as defined in claim 2, as well as by their addition salts with pharmaceutically acceptable salts.

9. Medicaments, characterised in that they are composed of the derivative of pyrazolobenzoxazine, as defined in claim 3, as well as by its addition salts with pharmaceutically acceptable acids.

10. Pharmaceutical compositions, characterised in that they contain, as active principle, at least one of the medicaments, as defined in one of claims 7, 8 or 9.

**Patentansprüche**

1. Pyrazolobenzoxazinderivate sowie deren Additionssalze mit Mineral- oder organischen Säuren, dadurch gekennzeichnet, daß sie der allgemeinen Formel I

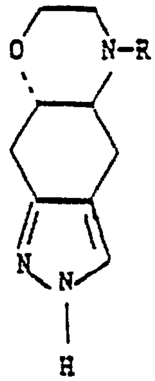

(I)

entsprechen, worin R für ein Wasserstoffatom oder einen Alkylrest mit 1 bis 5 Kohlenstoffatomen, einen Cycloalkylalkylrest mit 4 bis 7 Kohlenstoffatomen, einen Alkenyl- oder Alkinylrest mit 3 bis 5 Kohlenstoffatomen, mit der Maßgabe, daß sich die Mehrfachbindung nicht zwischen den Kohlenstoffatomen in α- und β-Stellung in bezug auf das Stickstoffatom befindet, oder einen Aralkylrest mit 7 bis 12 Kohlenstoffatomen steht, der gegebenenfalls durch einen oder mehrere Reste, ausgewählt unter den Alkylresten mit 1 bis 5 Kohlenstoffatomen, den Alkoxyresten mit 1 bis 5 Kohlenstoffatomen, der Aminogruppe, dem Hydroxyrest und den Halogenatomen, substituiert ist.

2. Pyrazolobenzoxazinderivate gemäß Anspruch 1 sowie deren Additionssalze mit Mineral- oder organischen Säuren, dadurch gekennzeichnet, daß R für ein Wasserstoffatom oder einen Alkylrest mit 1 bis 5 Kohlenstoffatomen steht.

3. Das Derivat der Formel I gemäß Anspruch 1 mit der folgenden Bezeichnung: (4a-SR-trans)-5-Propyl-2,4,4a,5,6,7,8a,9-octahydro-pyrazolo[4,3-g]-1,4-benzoxazin sowie dessen Additionssalze mit Mineral- oder organischen Säuren.

4. Verfahren zur Herstellung der Pyrazolobenzoxazinderivate der Formel I gemäß Anspruch 1 sowie von deren Salzen, dadurch gekennzeichnet, daß man das Enolat eines Ketons der Formel II

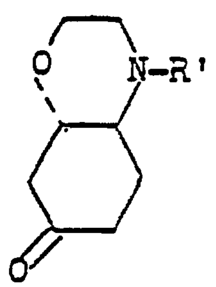

(II)

worin R′ die für R angegebene Bedeutung mit Ausnahme von Wasserstoff besitzt, mit einem Alkylformiat der Formel III

HCOO-Alk (III)

worin Alk für einen Alkylrest mit 1 bis 4 Kohlenstoffatomen steht, kondensiert, um zu einem Produkt der Formel IV

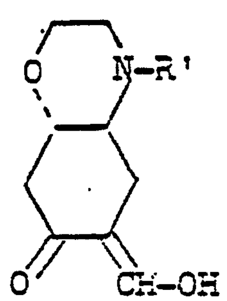

(IV)

zu gelangen, worin R′ die angegebene Bedeutung besitzt, das man mit Hydrazin umsetzt, um ein Produkt der Formel $I_A$

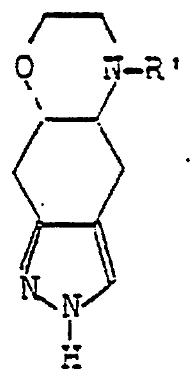

$(I_A)$

zu erhalten, worin R′ die angegebene Bedeutung besitzt, das man entweder isoliert und gewünschtenfalls in ein Salz überführt oder, wenn R′ für einen Benzylrest steht, einer katalytischen Hydrierung unterzieht, um zu einem Produkt der Formel $I_B$

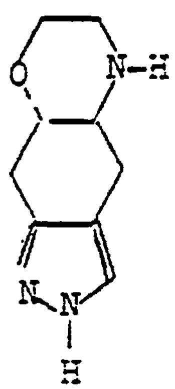

$(I_B)$

zu gelangen, das man entweder isoliert und gewünschtenfalls in ein Salz überführt, oder mit einem Halogenid der Formel V

Hal R″ (V)

umsetzt, worin Hal für ein Chlor-, Brom- oder Jodatom steht und R″ die für R angegebene Bedeutung mit Ausnahme von Wasserstoff und Methyl besitzt, um zu einem Produkt der Formel $I_C$

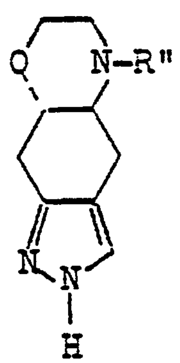

$(I_C)$

zu gelangen, worin R″ die angegebene Bedeutung besitzt, das man isoliert und gewünschtenfalls in ein Salz überführt.

5. Verfahren gemäß Anspruch 4, dadurch gekennzeichnet, daß das Alkylformiat das Ethylformiat ist; das Hydrazin in Form eines Hydrazinhydrats verwendet wird; der Hydrierungskatalysator Palladium ist; das Halogenid der Formel V ein Jodid ist.

6. Abänderung des Verfahrens gemäß Anspruch 4, dadurch gekennzeichnet, daß man das Keton der Formel II mit dem Dimethylformamid-dimethylacetal unter Rückfluß eines Lösungsmittels mit einem Siedepunkt von 80°C bis 130°C umsetzt, hiernach mit Hydrazin behandelt, um zu einem Produkt der Formel $I_A$ zu gelangen, das man gewünschtenfalls wie vorstehend angegeben behandelt.

7. Arzneimittel, dadurch gekennzeichnet, daß sie aus den neuen Pyrazolobenzoxazinderivaten der Formel I gemäß Anspruch 1 sowie aus deren Additionssalzen mit pharmazeutisch verträglichen Säuren bestehen.

8. Arzneimittel, dadurch gekennzeichnet, daß sie aus den neuen Pyrazolobenzoxazinderivaten gemäß Anspruch 2 sowie aus deren Additionssalzen mit pharmazeutisch verträglichen Säuren bestehen.

9. Arzneimittel, dadurch gekennzeichnet, daß sie aus dem Pyrazolobenzoxazinderivat gemäß Anspruch 3 sowie aus dessen Additionssalzen mit pharmazeutisch verträglichen Säuren bestehen.

10. Pharmazeutische Zusammensetzungen, dadurch gekennzeichnet, daß sie als Wirkstoff zumindest eines der Arzneimittel gemäß einem der Ansprüche 7, 8 oder 9 enthalten.